# EUROPÄISCHE PATENTSCHRIFT

(11) **EP 2 149 051 B1**
(45) Veröffentlichungstag und Bekanntmachung des Hinweises auf die Patenterteilung: **18.04.2012**
(21) Anmeldenummer: 08748781.5
(22) Anmeldetag: 25.04.2008
(51) Int. Cl.: G01N 33/564

(54) **VERFAHREN UND IMMUNABSORBENTIEN ZUR SPEZIFISCHEN DETEKTION UND ABSORPTION ZÖLIAKIE- UND DERMATITIS HERPETIFORMIS ASSOZIIERTER ANTIKÖRPER**
METHOD AND IMMUNE ABSORBENTS FOR THE SPECIFIC DETECTION AND ABSORPTION OF CELIAC DISEASE- AND HERPETIFORM DERMATITIS-ASSOCIATED ANTIBODIES
PROCÉDÉ ET AGENTS D'IMMUNOABSORPTION PERMETTANT LA DÉTECTION SPÉCIFIQUE ET L'ABSORPTION D'ANTICORPS ASSOCIÉS À LA MALADIE COELIAQUE ET À LA DERMATITE HERPÉTIFORME

(30) Priorität: 30.05.2007 DE 102007025291
(43) Veröffentlichungstag der Anmeldung: 03.02.2010
(73) Patentinhaber: Euroimmun Medizinische Labordiagnostika AG, 23560 Lübeck (DE)
(72) Erfinder: PROBST, Christian, 23909 Ratzeburg (DE); SCHLUMBERGER, Wolfgang, 23627 Gross Grönau (DE); STÖCKER, Winfried, 23627 Gross Grönau (DE); DÄHNRICH, Cornelia, 23627 Gross Grönau (DE); KOMOROWSKI, Lars, 23909 Ratzeburg (DE); MOTHES, Thomas, 04109 Leipzig (DE)
(74) Vertreter: Weber, Martin
(86) Internationale Anmeldenummer: PCT/DE2008/000713
(87) Internationale Veröffentlichungsnummer: WO 2008/145084

(56) Entgegenhaltungen:
- WO-A-2005/105129
- SCHWERTZ ELKE ET AL: "Serologic assay based on gliadin-related nonapeptides as a highly sensitive and specific diagnostic aid in celiac disease" CLINICAL CHEMISTRY, Bd. 50, Nr. 12, Dezember 2004 (2004-12), Seiten 2370-2375, XP002503352 ISSN: 0009-9147 in der Anmeldung erwähnt
- MOYLE PETER M ET AL: "Method for the synthesis of multi-epitopic Streptococcus pyogenes lipopeptide vaccines using native chemical ligation" JOURNAL OF ORGANIC CHEMISTRY, Bd. 71, Nr. 18, September 2006 (2006-09), Seiten 6846-6850, XP002503754 ISSN: 0022-3263
- MOTHES THOMAS: "Deamidated gliadin peptides as targets for celiac disease-specific antibodies" ADVANCES IN CLINICAL CHEMISTRY, ACADEMIC PRESS, LONDON, GB, Bd. 44, 1. Januar 2007 (2007-01-01), Seiten 35-63, XP009108488 ISSN: 0065-2423

## Beschreibung

Die vorliegende Erfindung betrifft Fusionspeptide, die sich von Komponenten des Gliadins ableiten, Testreagenzien zum diagnostischen Nachweis von Antikörpern gegen diese Peptide und Verfahren zur Immunabsorption solcher Antikörper zur Therapie der Zöliakie und der Dermatitis herpetiformis.

### Stand der Technik:

Bei Patienten mit Gluten-Unverträglichkeit (Gluten-sensitive Enteropathie; Kleinkinder: Zöliakie, Erwachsene: einheimische Sprue; Zöliakie wird im Rahmen der vorliegenden Anmeldung auch altersunabhängig und synonym zu Gluten-sensitiver Enteropathie verwendet) wird durch Verzehr Gluten-haltiger Getreideprodukte eine Schädigung der Dünndarmschleimhaut hervorgerufen. Es kommt zu einer Zottenatrophie und zu funktionellen Störungen. Das klinische Bild ist geprägt von Diarrhoe und den Folgen der Malabsorption (insbesondere Gewichtsverlust, bei Kindern Wachstumsretardierung). Bei einigen Patienten mit Gluten-sensitiver Enteropathie besteht zusätzlich eine Dermatitis herpetiformis Duhring: eine chronische, mit Blasenbildung einhergehende Hauterkrankung, die aber auch für sich allein auftreten kann.

Einen wichtigen Beitrag zur Diagnose der Gluten-sensitiven Enteropathie und der Dermatitis herpetiformis Duhring liefert die Untersuchung zweier verschiedener Antikörper: Gegen Gliadin und gegen Gewebs-Transglutaminase. Sie sichert die klinische Diagnose ab und eignet sich darüber hinaus zur Verlaufskontrolle und zur Überwachung einer Gluten-freien Diät oder eines Gluten-Belastungstests. Eine spezifische Abtrennung dieser beiden Antikörper aus dem Plasma der Patienten kann einen positiven therapeutischen Effekt mit sich bringen.

Während der akuten Krankheitsphase der Gluten-sensitiven Enteropathie sowie bei Dermatitis herpetiformis sind meist Gliadin-Antikörper der Klassen IgA und IgG nachweisbar. Antikörper der Klasse IgM sind gelegentlich auch nachweisbar, in der Regel aber nur, wenn gleichzeitig auch Antikörper gegen Gliadin der Klassen IgA und IgG vorliegen.

Mit Beginn einer Gluten-freien Diät fallen die IgA-Antikörper gegen Gliadin innerhalb weniger Monate auf niedrige Werte ab, während Antikörper der Klasse IgG im allgemeinen länger persistieren. Permanent hohe IgA-Gliadin-Antikörperspiegel sprechen dafür, daß eine Gluten-freie Diät nicht eingehalten wird. Unter Gluten-Belastung kommt es im Falle eines Rezidivs innerhalb weniger Tage zu einem Anstieg der IgG-Antikörper gegen Gliadin, die IgA-Antikörper folgen etwas später.

Die physiologische Funktion der Gewebs-Transglutaminase besteht in der Katalyse der Ausbildung einer Isopeptid-Bindung zwischen einer γ-Carboxamid-Gruppe eines Glutamin- und der ε-Aminogruppe eines Lysinrestes extrazellulärer Matrixproteine, wodurch diese kreuzvernetzt werden (Aeschlimann et al. 2000 Connective Tissue Res. 41:1-27; Gentile et al. 2002. Neurochem Int. 40:79-83).

Mit der Nahrung aufgenommene Glutamin-reiche Gliadine können nach der Resorption ebenfalls durch Gewebstransglutaminase modifiziert werden. Allerdings erfolgt hier vorzugsweise die enzymatische Desamidierung von Glutamin- zu Glutamat-Resten (zusammengefasst in Schwertz et al. 2004. Clinical Chemistry 50:2370-2375). Diese führt zu einer stärkeren Bindung von Gliadin-Peptiden an MHC-Klasse II Moleküle und dadurch zu einer gesteigerten T-Zell-Stimulation bei Patienten mit Zöliakie (Sjöström et al. 1998. Scand J Gastroenterol. 48:111-115; Molberg et al. 1998. Nat Med. 4:713-717).

Als wichtiges Epitop für Antikörper der Klasse IgA, insbesondere von Teilen des desamidierten Gliadins, wurde das Tripeptid Prolin-Glutamat-Glutamin (PEQ) identifiziert (Osman et al. 2000. Clin Exp Immunol. 121:248-254). Es wurde gezeigt, dass desamidiertes Gliadin spezifischer durch Antikörper von Zöliakie-Patienten erkannt wird als das unmodifizierte Gliadin (Aleanzi et al. 2001. Clin Chem. 47:2023-2028). Durch die Autoren wurde für den Nachweis von Antikörpern gegen ein desamidiertes, vom Gliadin abgeleitetes Peptid bei Zöliakie für Antikörper der Klasse IgA eine Sensitivität von 85,0% bei einer Spezifität von 86,7% und für Antikörper der Klasse IgG eine Sensitivität von 90,0% bei einer Spezifität von 86,7% hinsichtlich einer Zöliakie-Erkrankung ermittelt (siehe **Tabelle 1,a**).

Im Rahmen einer systematischen Analyse der Bindung Zöliakie-assoziierter Antikörper der Klasse IgA an synthetische, vornehmlich vom Gliadin abgeleitete Antigene wurden zwei Nonapeptide entsprechend der SEQ-ID NO 2 (Pro Leu Gln Pro Glu Gln Pro Phe Pro) und SEQ-ID NO 3 (Pro Glu Gln Leu Pro Gln Phe Glu Glu) als bestgeeignete Antigene identifiziert (Schwertz et al. 2004. Clinical Chemistry 50:2370-2375). Die Synthese dieser beiden Peptide (nach Kramer et al. 1998. J. Methods Mol. Biol. 87:25-39) erfolgte separat nebeneinander chemisch auf einer Zellulosemembran, die anschließend in einem Chemilumineszenztest direkt zur Bindung Zöliakie-assoziierter Antikörper eingesetzt wurde. Das Verfahren ist von der Herstellung Membranbasierter Peptid-Banken im Rahmen von Epitopmappingstudien bekannt. Entsprechende Reagenzien für den Antikörpernachweis führen bei der Produktion diagnostischer Testsysteme zu hohen Herstellungskosten.

Dessen ungeachtet haben Schwertz et al. zwei Membran-basierte ELISA zum Nachweis von Antikörpern der Immunglobulinklasse IgA gegen Gliadin bei Zöliakie mit Chemilumineszenz-Detektion durchgeführt, die in der kombinierten Auswertung beider Antigene entsprechend SEQ ID NO 2 und SEQ ID NO 3 zusammen (siehe **Tabelle 1**, **b**) eine Sensitivität von 94,2% bei einer Spezifität von 93,4% aufwiesen.

Die Chemilumineszenzdetektion ist aufwändig und auf teure Analysegeräte angewiesen. Es wurde nicht gezeigt, ob die erhobenen diagnostischen Leistungsdaten auch mit den mehrheitlich verbreiteten kolorimetrischen Detektionsverfahren zu erzielen sind. Die Autoren selbst weisen auf diesen Nachteil hin und schlagen zur weiteren Verbesserung ihres Testsystems eine erneute Sequenzoptimierung sowie die Überführung des Membranbasierten Chemilumineszenztestsystems auf das Mikrotiterplattenformat vor, ohne diese Aufgabe gelöst zu haben. Schwertz et al. haben nur Gewebstransglutaminase-IgA-positive Seren in die Studie einbezogen. Proben von Patienten mit selektivem IgA-Mangel führen bei Schwertz et al. nicht zu einer Verringerung der Sensitivität wie bei ergebnisoffener Patientenauswahl, sodass hier eine ungerechtfertigt zu hohe Sensitivität in der Größenordnung von 3-5% anzunehmen ist. Die Leistungsdaten basieren außerdem auf einer kombinierten Auswertung der mit zwei separaten und auf den Peptidsequenzen nach SEQ ID NO 2 bzw. SEQ ID NO 3 basierenden, schlecht routinetauglichen Bestimmungen.

Ein Mikrotiterplatten-basierter ELISA zur Bestimmung von Antikörpern der Klasse IgG auf Basis eines (gemäß der Sequenz SEQ ID NO 2) der beiden Nonapeptide wies in einer Studie (**Prause et al**. **2007**, Posterbeitrag zur 22. Jahrestagung der Gesellschaft für pädiatrische Gastroenterologie und Ernährung, Bochum) ebenfalls eine Verbesserung gegenüber dem Stand der Technik für die Bestimmung von Antikörpern der Klasse IgG auf, für IgA wurden keine Ergebnisse gezeigt (siehe **Tabelle 1**, **c**).

In der Studie von Schwertz et al. hat sich gezeigt, dass die getrennt durchgeführte aber kombiniert ausgewertete Antikörperbestimmung unter Einsatz der Peptide gemäß SEQ ID NO 2 und SEQ ID NO 3 eine Verbesserung der serologischen Zöliakie-Diagnostik ermöglicht. Die Leistungsdaten der Arbeit von Aleanzi et al. wurden von Schwertz et al. deutlich übertroffen. Bei Schwertz et al. müssen aber mindestens zwei getrennte Synthesen und Bestimmungen (Antigene entsprechend SEQ ID NO 2 und SEQ ID NO 3) separat durchgeführt werden, was den doppelten Material- und Arbeitsaufwand verursacht. Es wäre möglich, die beiden Peptidantigene zu mischen und das Gemisch zur Beschichtung der Festphase im ELISA einzusetzen. Solche Tests leiden aber an mangelnder Reproduzierbarkeit, einer Zunahme der unspezifischen Reaktionen und zu gegenseitiger sterischer Behinderung bei der Bindung der antigenen Komponenten an die Oberfläche.

**Tabelle 1: Zusammenstellung der publizierten Leistungsdaten von Testsystemen basierend auf desamidierten Gliadin-homologen Peptidantigenen.**

| | Immunglobulin-Klasse | Sensitivität | Spezifität | Quelle |
|---|---|---|---|---|
| a | IgA | 85,0% | 86,7% | Aleanzi et al., 2001 SEQ ID NO 1 |
| | IgG | 90,0% | 86,7% | |
| b* | IgA | ≤ 94,2% | 93,4% | Schwertz et al., 2004 SEQ ID NO 2 und SEQ ID NO 3 |
| | IgG | nicht ausreichend? | nicht ausreichend? | |
| c | IgA | nicht ausreichend? | nicht ausreichend? | Prause et al., 2007 SEQ ID NO 2 |
| | IgG | 95,0% | 94,7% | |

| | | | | |
|---|---|---|---|---|
| * inkorrekte vorselektierte Patientenauswahl | | | | |

Aufgabe der Erfindung war es, die oben beschriebenen Nachteile des Standes der Technik zu überwinden und ein besser routinetaugliches Antikörper-Testsystem für die Diagnose der Zöliakie bereitzustellen, welches kompetente Resultate liefert.

### Beschreibung der Erfindung:

Diese Aufgabe wird durch den Gegenstand der Ansprüche, insbesondere durch die im Folgenden beschriebenen Fusionspolypeptide gelöst.

Aus dem Stand der Technik lässt sich ableiten, das mit Hilfe der Einzelpeptide nach SEQ ID NO 2 und SEQ ID NO 3 eine kompetente Diagnose der Zöliakie möglich ist. Wir synthetisierten diese beiden Peptide deswegen zunächst chemisch in biotinylierter Form und setzten sie zur Beschichtung von Mikrotiterplatten ein. Durch den Nachweis gebundenen Biotins konnte sichergestellt werden, dass die Peptide sich an die Oberfläche gebunden hatten, allerdings gelang es nicht, die aus dem Stand der Technik abgeleiteten diagnostischen Leistungsparameter nachzuvollziehen. Daraufhin stellten wir die Versuche mit den chemisch synthetisierten Peptiden gemäß SEQ ID NO 2 und SEQ ID NO 3 ein.

Als Alternative zur chemischen Synthese wäre es möglich gewesen, die Peptide gemäß SEQ ID NO 2 und SEQ ID NO 3 rekombinant zu exprimieren (siehe Beispiele 1 und 2), und zwar in der Form mehrerer fusionierter, jeweils durch Methioninreste voneinander getrennte Kopien, im Bereich derer mittels Bromcyan gespalten werden könnte, um sie zu vereinzeln. Dann hätte man die Komponenten aufgetrennt, jeweils einzelne ELISA durchgeführt und parallel für die Diagnostik eingesetzt oder man hätte mit der Mischung beschichtet.

Die Erfinder haben aber die Idee verfolgt, die Peptide gemäß SEQ ID NO 2 und SEQ ID NO 3 zu einem neuen Fusionspeptid gemäß SEQ ID NO 4 (Pro Leu Gln Pro Glu Gln Pro Phe Pro Glu Gln Leu Pro Gln Phe Glu Glu) zusammenzusetzen. Von diesem Fusionspeptid sollten mehrere Kopien aneinandergefügt synthetisiert und nach erfolgter rekombinanter Synthese die Peptide wieder vereinzelt werden. Wir synthetisierten eine entsprechende DNA und integrierten sie in trimerer Form in ein *E.coli-*Expressionsplasmid, sodass wir das zirkuläre Plasmid gemäß SEQ ID NO 6 erhielten (siehe Beispiel 1). Mit Hilfe dieses Plasmids konnten wir das im Folgenden als Trimer bezeichnete Polypeptid gemäß SEQ ID NO 7 in *E*. *coli* exprimieren, aufreinigen (siehe Beispiel 2) und anschließend, wie erwartet, durch Behandlung mit Bromcyan spalten. Das Spaltprodukt (Monomer) setzten wir zur Beschichtung von Mikrotiterplatten ein. Parallel dazu führten wir Kontrollen mit dem ungespaltenen Trimer unter identischen Bedingungen durch. Überraschenderweise erzielten wir im ELISA mit kolorimetrischer Detektion (siehe Beispiel 3) mit dem Trimer als Antigensubstrat wesentlich bessere Leistungsdaten für die Immunglobulinklasse IgG zur Identifizierung der Zöliakie-Patienten als mit Testsystemen des bereits hoch entwickelten Standes der Technik (**siehe Tabelle 2**).

Auch mit Monomeren, die durch Spaltung aus dem Trimer gewonnenen wurden, ließen sich in Vorversuchen die Leistungsdaten der ELISA-Systeme des Standes der Technik bereits übertreffen, die weitaus besten Ergebnisse wurden aber mit dem ungespaltenen Trimer erreicht. Peptidantigene aus mehr als 3 Polypeptidsequenzen gemäß SEQ ID NO 4 können ebenfalls überragende Leistungsdaten erzielen

Zur Ermittlung der Leistungsdaten des ELISA basierend auf dem Polypeptid gemäß SEQ ID NO 7 (Trimer) führten wir im Folgenden eine Studie mit Serumproben von Personen mit bioptisch gesicherter positiver bzw. negativer Zöliakiediagnose durch (siehe Beispiel 3). Im Unterschied zu Schwertz et al. wurde das Kollektiv nicht vorselektiert, d.h. Patienten mit selektivem IgA-Mangel wurden nicht ausgeschlossen. Das Kollektiv setzte sich folgendermaßen zusammen:
Gruppe 1: Zöliakiepatienten ohne spezielle Diät zum Zeitpunkt der Biopsie (Marsh 2-3). Diese Gruppe umfasst auch Patienten mit Dermatitis herpetiformis.
Gruppe 2: Patienten mit Dermatitis herpetiformis, bei denen keine Zöliakie bekannt ist.
Gruppe 3: Patienten, bei denen die bioptische Untersuchung gegen eine Zöliakie spricht (Marsh 0).
Gruppe 4: Patienten mit chronisch-entzündlichen Darmerkrankungen (Morbus Crohn und Colitis ulcerosa), bei denen eine dünndarmbioptische Untersuchung erfolgte und in der keine zöliakietypische Veränderungen beschrieben wurden.

Die Gruppe 1 wurde als klinisch gesicherte Zöliakie-Positiv-, die Gruppe 2 als klinisch gesicherte Dermatitis herpetiformis-Positiv- und die Gruppen 3 und 4 als Negativkontrollgruppen zur Ermittlung der Leistungsparameter durch Signal-Analyse mittels "Receiver Operating Characteristic"-Kurven (ROC)-Analyse eingesetzt.

Ergebnisse: Wie in **Tabelle 2** wiedergegeben, zeigte sich, dass der ELISA basierend auf dem Polypeptid gemäß SEQ ID NO 7 eine höhere Sensitivität und Spezifität für den Nachweis Zöliakie-spezifischer Antikörper der Klasse IgG aufweist als der von Prause et al. beschriebene Test. Darüber hinaus ist mit dem gleichen erfindungsgemäßen ELISA auch eine qualitativ hochwertige Bestimmung von Antikörpern der Klasse IgA möglich.

Wir konnten zeigen, dass sich durch das Zusammenfügen der Peptidsequenzen gemäß SEQ ID NO 2 und SEQ ID NO 3 zu einem Peptid gemäß SEQ ID NO 7 Antigene herstellen lassen, die sich als Substrate für robuste, einfach zu produzierende ELISA mit kolorimetrischer Detektion eignen. Mit Testsystemen, die Antigensubstrate gemäß der Erfindung enthalten, können Zöliakieassoziierte Antikörper gegenüber dem Stand der Technik einfacher, sicherer und mit höherer Genauigkeit bestimmt werden. Darüberhinaus ermöglicht das erfindungsgemäße Verfahren auch die spezifische Bestimmung von Antikörpern der Klassen IgA und IgG bei Patienten mit Dermatitis herpetiformis (siehe **Tabelle 2**).

**Tabelle 2: Leistungsparameter des erfindungsgemäßen ELISA hinsichtlich der Diagnose einer Zöliakie bzw. einer isolierten Dermatitis hepetiformis.**

| | | Sensitivität | Spezifität |
|---|---|---|---|
| Zöliakie | IgA | 80,8% | 94,6% |
| | IgG | 98,0% | 96,0% |
| Dermatitis herpetiformis | IgA | 66,7% | 94,6% |
| | IgG | 66,7% | 96,0% |

In Bezug auf die Diagnostik der Zöliakie erzielten wir mit einem Reagenz entsprechend der Erfindung (Variante SEQ ID NO 7, Trimer) für den Nachweis Zöliakie-assoziierter Antikörper der Klasse IgG eine Sensitivität von 98,0% bei einer Spezifität von 96,0%. Diese Werte liegen deutlich über den bisher besten publizierten Daten (Prause et al.: Sensitivität 95,0%, Spezifität 94,7%).

Was Antikörper der Klasse IgA betrifft, beträgt die Sensitivität 80,8% und liegt damit unter den von Aleanzi et al. und Schwertz et al. (siehe **Tabelle 1 a und b**) publizierten Daten. Aleanzi et al. haben jedoch eine ungenügende Spezifität von 86,7% erzielt und liegen daher, Spezifität und Sensitivität zusammen genommen, hinsichtlich des IgA gleichauf mit unseren Ergebnissen. Die Werte von Schwertz et al. können außerdem nur bedingt zum Vergleich herangezogen werden, weil das Patientenkollektiv vorselektioniert war. Der Verzicht auf Patientenproben mit selektivem IgA-Mangel (ca. 3% der an Zöliakie Erkrankten; Challacombe et al. 1995, Arch. Dis. Childhood 73:3-7; siehe Anmerkung zu Tabelle 1) spiegelt bei Schwertz et al. eine zu hohe diagnostische Sensitivität vor, die in einem nicht vorselektierten Kollektiv so nicht zu reproduzieren ist. Außerdem ist das Testsystem von Schwertz et al. nicht für eine industrielle Produktion einsetzbar.

Für die Immunglobulinklasse IgG wurde die Überlegenheit eines der Zielantigene gemäß der Erfindung gegenüber dem Stand der Technik eindeutig bewiesen. Da nach aller Erfahrung bei Zöliakie und Dermatitis herpetiformis die Antikörper gegen Gliadin der Immunglobulinklassen IgA und IgG gegen gleiche Antigenepitope gerichtet sind, ist davon auszugehen, dass auch für IgA bei Verwendung des Probenmaterials identischer Patientenkollektive in den Testsystemen des Standes der Technik und der vorliegenden Erfindung für letztere die besten Leistungsparameter ermittelt werden können.

Antigene gemäß der Erfindung sind technisch einfach, in ausreichender Menge und bester Qualität herstellbar. Sie lassen sich ohne weiteres an definierte Oberflächen binden. Sie eignen sich, unter anderem, aber nicht ausschließlich, für Festphasenimmuntests und immunaffinitätsschromatographische therapeutische Verfahren.

Gegenstand der Erfindung ist ein Polypeptid, das ein oder mehrere Peptide entsprechend SEQ ID NO 2 und ein oder mehrere Peptide entsprechend SEQ ID NO 3 in beliebiger Reihenfolge enthält, wobei das Polypeptid ein oder mehrere Polypeptide bestehend aus mindestens zehn, mindestens 14, mindestens 15 oder mindestens 16 in SEQ ID NO 4 aufeinanderfolgenden Aminosäuren umfasst oder ein oder mehrere Polypeptide gemäß SEQ ID NO 4 umfasst. Dabei können an beliebiger Stelle bis zu 30% der Aminosäuren entfernt oder gegen beliebige Aminosäuren ausgetauscht sein und/oder sich SEQ ID NO 2 und SEQ ID NO 3 zum Teil überschneiden, wobei die Aktivität des Polypeptids vergleichbar bleiben sollte, insbesondere die Bindung von Zöliakie- und Dermatitis herpetiformis-spezifischen Antikörpern. Insbesondere kann das Polypeptid 1, 2, 3, 4 oder 5 Aminosäureaustausche, insbesondere konservative Aminosäureaustausche, oder Deletionen enthalten (verteilt auf beide Sequenzen, insbesondere 1, 2, oder 3 Aminosäureaustausche oder Deletionen pro Sequenz.

Selbstverständlich kann das Polypeptid auch weitere Sequenzen enthalten. In einer Ausführungsform ist mehr als 50% des Peptids einer der Sequenzen nach SEQ ID NO 2 oder 3 zuzuordnen. Insbesondere können bis zu 30% oder bis zu 40% beliebiger Aminosäuren hinzugefügt werden. Diese können z.B. einer leichteren Aufreinigung des Peptids (z.B. His-Tag, Flag-Tag o.ä.), einer besseren Stabilität oder einer leichteren Zugänglichkeit für Antikörper dienen (z.B. als Spacer zwischen den Epitop-Sequenzen). Selbstverständlich können auch weitere für die Diagnose oder Therapie von Zöliakie und/oder Dermatitis hepatiformis nützliche Epitope, z.B. aus Gewebstransglutaminase, enthalten sein.

Die Polypeptide können alternativ auch nur eine Kopie der jeweiligen Peptidsequenzen enthalten, insbesondere enthalten sie aber Peptidsequenzen entsprechend SEQ ID NO: 2 und SEQ ID NO 3, d.h., jeweils eine Mehrzahl an Kopien. Bevorzugt umfassen solche Polypeptide zwei oder mehr, insbesondere drei oder mehr oder drei bis zwanzig oder 4 bis 10 Kopien der jeweiligen Sequenzen. Dabei kann die Anzahl der Kopien von SEQ ID NO 2 und SEQ ID NO 3 gleich oder auch unterschiedlich sein, und die Sequenzen können sich zum Teil überschneiden, müssen dies aber nicht. Zum Beispiel können 2 Kopien von SEQ ID NO 2 und 3 Kopien von SEQ ID NO 3 vorliegen oder umgekehrt.

Die kleinste Einheit des bevorzugten erfindungsgemäßen Polypeptids enthält die durch Schwertz et al. beschriebenen Peptide gemäß SEQ ID 2 und SEQ ID 3 in einer sich überschneidenden Sequenz gemäß SEQ ID NO 4.

Es müssen daher im Vergleich zu Schwertz et al. keine zwei Tests parallel angesetzt werden. Multimere Formen der erfindungsgemäßen Einheit, insbesondere der kleinsten Einheit bieten, nachdem sie an eine Oberfläche gekoppelt wurden, eine höhere Sicherheit als monomere Formen, da ausreichend Epitope für die Reaktion mit den spezifischen Antikörpern zur Verfügung stehen und immer noch welche übrig bleiben, wenn Bereiche des Polypeptids für die Bindungsreaktion an die Oberfläche unzugänglich geworden sind.

Der in der vorliegenden Erfindung gegenüber dem Stand der Technik nochmals erreichte Qualitätssprung ist wahrscheinlich am größten bei den neuen Peptidsequenzen gemäß SEQ ID NO 4 bzw. SEQ ID NO 7. Diese Peptidsequenzen sind gegenüber dem Stand der Technik als neue Qualität anzusehen. Die Polypeptide mit mehr oder weniger als drei Kopien des Peptids gemäß SEQ ID NO 4, insbesondere mit 2 oder mehr, drei oder mehr oder 4 oder mehr Kopien, sowie von Teilpeptiden bestehend aus mindestens zehn, mindestens 14, mindestens 15 oder mindestens 16 in SEQ ID NO 4 aufeinanderfolgenden Aminosäuren, lassen sich wie das Polypeptid gemäß SEQ ID NO 7 einsetzen und zeigen bevorzugt eine vergleichbare Bindung der Antikörper.

Für Polypeptide aus den in beliebiger Reihenfolge und Anzahl zusammengesetzten Polypeptiden gemäß SEQ ID NO 2 und SEQ ID NO 3, sowohl mit als auch ohne Sequenzüberschneidungen, sind mit dem Fusionspolypeptid gemäß SEQ ID NO 4 vergleichbare Eigenschaften hinsichtlich der Bindung Zöliakie- und Dermatitis herpetiformis spezifischer Antikörper zu erwarten.

Dem Fachmann ist bekannt, dass sich physikochemisch ähnliche Derivate aus den bisher beschriebenen Polypeptiden durch eine oder mehrere konservative Aminosäureaustausche (Glutamat zu Aspartat E->D, Glutamin zu Asparagin Q->N, Phenylalanin zu Tyrosin F->Y, Leucin zu Isoleucin L->I) an beliebigen Positionen generieren lassen. Auch solche Derivate sind Gegenstand der Erfindung und im Rahmen der Erfindung immer mit eingeschlossen, da vergleichbare Eigenschaften hinsichtlich der Bindung Zöliakie- und Dermatitis herpetiformis spezifischer Antikörper zu erwarten sind. Unter vergleichbaren Eigenschaften versteht der Fachmann eine ähnliche Spezifität und Sensitivität, insbesondere für den diagnostischen Nachweis von Zöliakie und/oder Dermatitis herpetiformis wie in Tabelle 2 für den Nachweis von IgA und/oder von IgG angegeben (- 2% oder besser oder bevorzugt - 1% oder besser), insbesondere ist die Bindung der Antikörper also mit der Bindung an das Fusionspolypeptid nach SEQ ID NO: 7 vergleichbar. Insbesondere können bis zu 10%, bis zu 20% oder bis zu 30% der Aminosäuren durch einen oder mehrere konservative Aminosäureaustausche ausgetauscht sein, wobei die Bindung von Zöliakie und Dermatitis herpetiformis spezifischen Antikörpern vergleichbar ist.

In einer bevorzugten Ausführungsform enthält ein erfindungsgemäßes Polypeptid mindestens eine und maximal 20 Kopien der Sequenz gemäß SEQ ID NO 4. In einer besonders bevorzugten Ausführungsform enthält ein erfindungsgemäßes Polypeptid mindestens zwei und maximal zehn Kopien der Sequenz gemäß SEQ ID NO 4 oder mindestens drei Kopien der Sequenz gemäß SEQ ID NO: 4. In einer anderen Ausführungsform wird ein Gemisch unterschiedlicher erfindungsgemäßer Polypeptide eingesetzt.

Die erfindungsgemäßen Polypeptide können auch in der Form von Polymeren vorliegen, insbesondere Polymere, die 2 bis 20 oder 3 bis 10 erfindungsgemäße Polypeptide umfassen. Diese müssen nicht durch eine Peptidbindung miteinander verbunden sein, sondern können auch auf andere Weise (z.B. über eine Verbindungsgruppe) 1 kovalent gekoppelt oder nichtkovalent assoziiert sein.

Für den Fachmann ergeben sich weitere Verfahren zur Herstellung, Aufreinigung und Isolierung der erfindungsgemäßen rekombinanten Polypeptide, mit oder ohne Fusionspartner, wie etwa Filtrations-, Chromatographie-, Elektrophorese- und Zentrifugationsverfahren oder Kombinationen aus diesen. Solche Verfahren zur Aufreinigung von Proteinen sind z.B. in Lottspeich und Zorbas beschrieben (Bioanalytik, 1998, Spektrum akademischer Verlag, Heidelberg, Berlin) oder Sambrook et al. (Molecular cloning: a laboratory manual, 2000, Cold Spring Harbor Laboratory Press, Cold Spring Harbor, NY). Gegenstand der Erfindung sind deshalb auch mit solchen Verfahren hergestellte, aufgereinigte oder isolierte erfindungsgemäße Polypeptide. In einer bevorzugten Ausführungsform wird das erfindungsgemäße Polypeptid in *E*. *coli* rekombinant hergestellt.

Gegenstand der Erfindung sind daher auch Nukleinsäuren (DNA oder RNA), welche für ein oder mehrere erfindungsgemäße Fusionspolypeptide kodieren, z.B. für ein Polypeptid umfassend das Peptid gemäß SEQ ID NO 4 oder SEQ ID NO 7. Diese können in einem Expressionsvektor unter Kontrolle eines heterologen Promotors vorliegen. Der Expressionsvektor kann in einer Wirtszelle enthalten sein. Es hat sich im Rahmen der Erfindung herausgestellt, dass z.B. *E*. *coli* zur Expression der erfindungsgemäßen Fusionspolypeptide geeignet ist.

Gegenstand der Erfindung sind auch mit einem Reportermolekül markierte erfindungsgemäße Polypeptide. Als chemische Kopplungsverfahren sind dem Fachmann eine Reihe unterschiedlicher Verfahren wie solche, die die Reportermoleküle zunächst mit einer reaktiven Gruppe, bspw. Pentafluorophenylester, N-Hydroxysuccinimid oder Maleimid, versehen und die so aktivierten Reportermoleküle dann mit dem zu markierenden Zielmolekül, bspw. einem Antigen oder Protein, in Kontakt bringen, bekannt. Nach der Verbindung von Reportermolekül und Zielmolekül kann überschüssiges Reportermolekül anschließend durch chemische, biochemische oder physikalische Trenntechniken, bspw. Chromatographie oder Filtration, entfernt werden. Als Reportermoleküle sind dem Fachmann eine Reihe unterschiedlicher Verbindungen wie Fluorophore, bspw. Fluorescein oder Tetramethylrhodamin, Haptene, bspw. Biotin oder His-Tag und Enzyme, bspw. Meerretich-Peroxidase oder alkalische Phosphatase, bekannt. Zur Nutzung der verschiedenen Kopplungsverfahren sind zu diesem Zweck bereits aktivierte Reportermoleküle kommerziell erhältlich. Der Fachmann kennt weitere Reportermoleküle und Kopplungsverfahren zur Markierung von Proteinen. Gegenstand der Erfindung sind deshalb auch mit solchen Verfahren hergestellte mit Reportermolekülen markierte erfindungsgemäße Polypeptide.

Gegenstand der Erfindung sind auch erfindungsgemäße Polypeptide, die mit einer festen Phase (z.B. einem Träger aus Plastik wie einer Mikrotiterplatte, einem magnetischen Kügelchen, einer Membran z.B. aus Nitrozellulose) direkt oder indirekt verbunden sind.

Gegenstand der Erfindung sind auch Verfahren zur Diagnose von Zöliakie und/oder Dermatitis herpetiformis, bei denen eine flüssige biologische Probe eines Patienten (insbesondere Blut, Plasma oder eine optional mit Wasser oder einem geeigneten Puffer verdünnte Stuhlprobe) mit erfindungsgemäßen Polypeptiden in Kontakt gebracht wird und die Bindung von Antikörpern aus der biologischen Probe an diese Polypeptide erfolgt oder erfolgen kann, wobei durch den Nachweis dieser Antikörper gegen eines dieser Polypeptide eine Zöliakie oder Dermatitis herpetiformis diagnostiziert wird. Bei diesen Verfahren kann ein Gemisch aus mindestens zwei verschiedenen erfindungsgemäßen Polypeptiden verwendet werden.

Im Rahmen der Erfindung wurde festgestellt, dass die erfindungsgemäßen Polypeptide besonders geeignet zur Durchführung immunologischer Verfahren wie etwa Westernblot, LinienBlot, Dot-Blot oder Immunfluoreszenztest sind. Gegenstand der Erfindung sind deshalb auch solche Verfahren, bzw. Diagnoseverfahren, bei denen die Bindung der Antikörper mit einem Immunfluoreszenztest, Microarray, ELISA, Lumineszenztest, Blot, Radioimmuntest, Western Blot oder Dot Blot nachgewiesen wird.

Die Erfindung stellt auch einen Testsatz zum Nachweis von Antikörpern zur Verfügung, der ein oder mehrere erfindungsgemäße Polypeptide umfasst. Dieser ermöglicht z.B. die Durchführung eines erfindungsgemäßen Diagnoseverfahrens. Ein solcher Testsatz kann auch sekundäre Antikörper enthalten, z.B. anti-human IgA und/oder anti-human IgG-Antikörper, die optional mit einem Reportermolekül markiert sein können. In einer Ausführungsform umfasst der Testsatz ferner Antigene, die zusätzlich zur Diagnose der Zöliakie und/oder Dermatitis herpetiformis geeignet sind, etwa Gewebstransglutaminase oder Polypeptide mit Epitopen daraus, die zur Diagnose geeignet sind. Damit kann der Testsatz z.B. zu einem kombinierten Test verwendet werden in dem IgA-Antikörper gegen Gewebstransglutaminase und IgG-Antikörper gegen die erfindungsgemäßen Polypeptide nachgewiesen werden.

Weiterer Gegenstand der Erfindung ist die therapeutische Verwendung der Polypeptide zur Entfernung Zöliakie-spezifischer Antikörper aus Blut oder Blutplasma, vorzugsweise von Patienten mit Zöliakie und Dermatitis herpetiformis. Dieses kann beispielsweise durch *ex vivo* Immunadsorption (Immunapherese) an immobilisierte erfindungsgemäße Polypeptide erfolgen (gemäß Beispiel 4). Durch In-Kontakt-bringen mit dem Blut oder Plasma werden die für die immobilisierten Polypeptide spezifischen Antikörper entfernt, und die Körperflüssigkeit kann anschließend wieder reinfundiert werden. Entsprechende Verfahren sind z.B. zur Therapie der dilatativen Kardiomyopathie auf Basis der Sequenz des beta-adrenergenen Rezeptors beschrieben (Rönspeck et al. 2003 Ther Apher Dial. 7:91-97). In einer bevorzugten Ausführungsform wird ein Polypeptid gemäß SEQ ID NO 7 durch chemische Kopplung an einer festen Matrix immobilisiert und als Grundlage für einen Immuntest oder ein therapeutisches immunaffinitätschromatographisches Absorptionsverfahren eingesetzt.

Die Erfindung stellt damit die Polypeptide zur Verwendung bei der Therapie von zöliakie und/oder Dermatitis herpetiformis bereit, bei dem Blut oder Plasma eines Patienten mit einem oder mehreren festphasengebundenen, erfindungsgemäßen Polypeptiden in Kontakt gebracht wird, die Bindung der krankheitsassoziierten Antikörper aus dem Blut oder Plasma an diese Polypeptide erfolgt, wobei das Antikörper-abgereicherte Blut oder Plasma zur Reinfusion an den Patienten geeignet ist. In einer Ausführungsform werden die erfindungsgemäßen Polypeptide hierbei in Kombination mit Polypeptiden mit Epitopen der Gewebstransglutaminase verwendet.

Die Erfindung betrifft auch die Verwendung eines oder mehrerer der beschriebenen Polypeptide zur Herstellung einer pharmazeutischen Zusammensetzung, insbesondere zur Therapie der Zöliakie und/oder Dermatitis herpetiformis. Diese pharmazeutische Zusammensetzung ist insbesondere zur Bindung /Absorption von Antikörpern verschiedener Klassen (IgA, IgG) aus Patientenblut oder -plasma geeignet.

MND00009256

### Beispiel 1: Klonierung von pET24e-(Gliadin-rd)₃

### Hestellung des Vektorfragmentes:

Zunächst wurde die unikale AdeI-Schnittstelle des Plasmides pET24d (Novagen) zerstört. Die Abtrennung der 3'-Einzelstrangüberhänge nach AdeI-Linearisierung des pET24d-Plasmides wurde durch PfU-Polymerase (Fermentas) in Abwesenheit von dNTP's katalysiert. Anschließend erfolgte die Religation des Vektorfragmentes durch T4-DNA-Ligase (Fermentas). Diese Plasmid-DNA wurde anschließend mit Hilfe der DNA-Oligonukleotidprimer sense pET24e (SEQ ID NO 8) und asense pET24e (SEQ ID NO 9) amplifiziert und nach AdeI-Restriktion und Dephosphorylierung der 5'-Enden als pET24e-Vektorfragment zum Einbau der für die vom partiell desamidierten Gliadin abgeleitete synthetische DNA-Sequenz eingesetzt.

Mit Hilfe der 5'-phosphorylierten DNA-Oligonukleotide sense dpGlia (SEQ ID NO 10) und asense dpGlia (SEQ ID NO 11) wurde eine Linkersequenz generiert, die AdeI-kompartible 5'-phosphorylierte Überhänge trägt und über diese gerichtet in das AdeI-geschnittene und dephosphorylierte pET24e-Vektorfragment mit Hilfe von T4-DNA-Ligase (Fermentas) ligiert.

Die Linker-Hybridisierung erfolgte im 50 µl Ansatz folgender Zusammensetzung:
75 mM Tris-HCl (pH 8.8 at 25°C)
20 mM (NH₄)₂SO₄
0.01 % (v/v) Tween 20;
2 mM MgCl₂
1 µM sense Glia-dp (SEQ ID NO 10)
1 µM asense Glia-dp (SEQ ID NO 11)

Der Ansatz wurde 1 Minute bei 95°C inkubiert und anschließend mit einer Temperaturerniedrigungsrate von 2 °C pro Minute auf 25°C gekühlt.

Die hybridisierten Linker-Fragmente wurden unter Verwendung des NucleoSpin^{®} Extract II-Aufreinigungssystems (MACHEREY-NAGEL GmbH & Co. KG) entsprechend den Angaben des Herstellers aufgereinigt und in jeweils 50 µl 5 mM Tris-HCl pH 8,5 aufgenommen.

Die Linker tragen beidseitig einen 3'-ATG-Überhang, wodurch ein gerichteter Einbau in den AdeI-geschnittenen pET24e-Vektor möglich ist.

Die 5'-phosphorylierten und aufgereinigten Linker-Fragmente wurden anschließend in AdeI-geschnittenes pET24e-Plasmid durch Ligation integriert. Zur Ligation wurde das Rapid DNA Ligation Kit der Firma Fermentas entsprechend den Angaben des Herstellers eingesetzt. Der Ligationsansatz wurde anschließend in *E.coli* BL21(DE3) (Stratagene) transformiert.

Positive Klone werden aufgrund der Kanamycin-Resistenz [50 µg/ml] selektiert. Aus diesen Klonen werden die enthaltenen Plasmide isoliert und durch Restriktionsanalyse und DNA-Sequenzierung überprüft. Die für das rekombinante Protein (Gliadin analoges Fusionspeptid GAF(3X)) nach SEQ ID NO 7 kodierende DNA-Sequenz ist in SEQ ID NO 5 und die DNA-Sequenz des gesamten Expressionsplasmides mit der Bezeichnung pET24e-GAF(3X) ist in SEQ ID NO 6 dargestellt. Korrekte Plasmide wurden für die Durchführung der Expression ausgewählt, in 20 ml antibiotikahaltigem LB-Medium inokkuliert und bei 37°C bis zum Erreichen einer OD₆₀₀ (d=l cm) von 0,6 inkubiert. Durch Zugabe von IPTG (Endkonzentration: 1 mM) wird die Proteinexpression induziert und anschließend für weitere 3 Stunden bei 37°C inkubiert. Nach dieser Zeit werden die Zellen durch Zentrifugation bei 2.200 x g für 10 Minuten sedimentiert, in 20 ml PBS resuspendiert, erneut zentrifugiert und schließlich in 1 ml PBS resuspendiert.

Der Zellaufschluss erfolgt durch Zugabe von einem Drittel Volumen 4 x NuPAGE-LDS-Probenpuffer (Invitrogen), gefolgt von einer 10 minütigen Inkubation bei 70°C. Chromosomale DNA wird anschließend durch Ultraschallbehandlung (Branson Sonifier, Stufe 7, MicroTip) fragmentiert.

Das Zelllysat wird nach der Trennung durch SDS-PAGE auf eine Nitrocellulose-Membran transferiert. Anschließend werden unabgesättigte Positionen der Membran durch eine 15 minütige Inkubation mit "Universalpuffer" (EUROIMMUN) mit 3% (w/v) Milchpulver blockiert. Anschließend wird 1 Stunde mit einem gegen den His-Tag gerichteten und 1:2000 in "Universalpuffer" mit 3% (w/v) Milchpulver verdünnten monoklonalen Antikörper aus der Maus (Merck Biosciences GmbH) inkubiert. Dann wird dreimal jeweils 5 Minuten mit "Universalpuffer" gewaschen. In einem zweiten Inkubationsschritt reagieren die im positiven Fall an die Proteine gebundenen Antikörper mit einer 1:2000 in "Universalpuffer" verdünnten Konjugat-Lösung, die als Konjugat ein alkalische Phosphatase-markierte Anti-Maus-IgG-Antikörper (Sigma) enthält. Anschließend wird wie nach der Seruminkubation gewaschen. In einem dritten Inkubationsschritt werden dann die gebundenen Antikörper mit einer NBT/BCIP-"SubstratLösung" (4-Nitrobluetetrazoliumchlorid / Chlorobromoindolylphosphate, EUROIMMUN) nachgewiesen.

Im Westernblot kann im Falle der Expression des (GAF)₃(His)₆-Konstruktes gemäß SEQ ID NO 7 ein His-Tag-reaktives Protein nachgewiesen werden, dessen Größe gut mit der auf Basis der Aminosäuresequenz vorhergesagten Masse von 7,9 kDa übereinstimmt. Zellen die den unveränderten Plasmidvektor enthalten, weisen kein entsprechendes Protein auf.

### Beispiel 2: Aufreinigung des rekombinanten trimeren Gliadin analogen Fusionspeptids GAF(3X) durch Affinitätschromatographie

Bei der Affinitätssäule handelt es sich um eine NiNTA-Spinsäule (Qiagen), die nach Empfehlungen des Herstellers gehandhabt wird.

Die gemäß Beispiel 1 geernteten Zellen werden erneut wie beschrieben zentrifugiert, in 1 ml "TNI-10-Puffer" (5 mM Tris-HCl pH 8,0, 300 mM NaCl, 10 mM Imidazol) resuspendiert und durch dreimalige, einminütige Ultraschallbehandlung (Branson Sonifier, Stufe 7, MicroTip) aufgeschlossen. Unlösliche Proteine werden durch Zentrifugation bei 21.250 x g für 10 Minuten abgetrennt. 0,5 ml des Überstandes anschließend auf eine mit "TNI-10-Puffer" äquilibrierte NiNTA-Spinsäule aufgebracht.

Nicht- oder schwachbindende Proteine werden durch mehrmaliges Waschen mit 0,5 ml "TNI-10-Puffer" von der Säule entfernt. Die Elution erfolgt mit 0,2 ml "TNI-150-Puffer" (5 mM Tris-HCl pH 8,0, 300 mM NaCl, 150 mM Imidazol). Die Elutionsfraktion wird durch Westernblot wie in Beispiel 1 analysiert.

Die Eluate enthalten im Wesentlichen ein Protein, das nach Färbung der Westernblotmembran mit Ponceau-S-Färbelösung jeweils einer Bande entsprechend einer Größe von 7,9 kDa entspricht. Nach Westernblot ist eine Bande derselben Größe zu sehen. Dieses Ergebnis deutet darauf hin, dass das exprimierte Protein nach der Chromatographie im Wesentlichen frei von *E*. *coli*-Bestandteilen vorliegt.

### Beispiel 3: ELISA zur Bestimmung von Zöliakie- und Dermatitisherpetiformis-spezifischen Antikörpern.

Die ELISA-Mikrotiterplatten (Maxisorb, Nunc) werden mit in PBS verdünntem Antigen über Nacht bei Raumtemperatur beschichtet. Als besonders bevorzugtes Antigen nutzten wir ein rekombinantes Fusionspolypeptid, zusammengesetzt aus einem immunologisch dominanten, desamidierten Nonapeptid des Gliadins, verknüpft mit einem künstlichen, Gliadin-homologen Oktapeptid ein (gemäß SEQ ID NO 7).

Anschließend werden die mit Antigen beschichteten Platten mit PBS(0,1% Tween 20) gewaschen und für 2 Stunden bei Raumtemperatur mit PBS-10% fetalem Rinderserum geblockt. Danach werden die Mikrotiterplatten erneut mit PBS-0,1% Tween 20 gewaschen. Die Inkubation der Platten erfolgt wie in den EUROIMMUN-Arbeitsanleitungen beschrieben. Die Ergebnisse zur Leistungsbewertung dieses ELISA sind in **Tabellen 2** zusammengestellt.

### Beispiel 4: Immuntherapeutisches Verfahren zur Behandlung der Zöliakie und der Dermatitis herpetiformis.

Aufgereinigtes Antigen gemäß der Erfindung wird an eine feste Phase gekoppelt und als Immunnaffinitätschromatographisches Säulenmaterial eingesetzt. Plasma eines Patienten mit Zöliakie oder Dermatitis herpetiformis wird im Durchfluss über diese Säule geleitet und dem Patienten Antikörper-abgereinigt wieder zugeführt.

## Patentansprüche

1. Polypeptid, das ein oder mehrere Peptide gemäß SEQ ID NO: 2 und ein oder mehrere Peptide gemäß SEQ ID NO: 3 umfasst, wobei jeweils bis zu 30 % der Aminosäuren entfernt oder durch einen konservativen Aminosäureaustausch ausgetauscht sein und/oder sich SEQ ID NO: 2 und SEQ ID NO: 3 zum Teil überschneiden können, **dadurch gekennzeichnet, dass** es ein oder mehrere Polypeptide bestehend aus mindestens 14, mindestens 15 oder mindestens 16 in SEQ ID NO: 4 aufeinanderfolgende Aminosäuren umfasst oder ein oder mehrere Polypeptide gemäß SEQ ID NO: 4 umfasst.

2. Polypeptid nach Anspruch 1, umfassend drei Polypeptide gemäß SEQ ID NO: 4.

3. Polypeptid nach einem der vorhergehenden Ansprüche, umfassend ein oder mehrere Polypeptide gemäß SEQ ID NO: 7.

4. Polypeptid nach einem der vorhergehenden Ansprüche in Form eines Polymers, umfassend 2 bis 20 Polypeptide.

5. Polypeptid nach einem der vorhergehenden Ansprüche, **dadurch gekennzeichnet, dass** es rekombinant oder mit herkömmlichen biochemischen Mitteln der Polypeptidsynthese hergestellt wird.

6. Polypeptid nach einem der vorhergehenden Ansprüche, **dadurch gekennzeichnet, dass** es mit einem Reportermolekül oder mit einer festen Phase verbunden ist.

7. Verfahren zur Diagnose von Zöliakie und/oder Dermatitis herpetiformis, **dadurch gekennzeichnet, dass** eine flüssige biologische Probe eines Patienten mit einem oder mehreren Polypeptiden nach einem der vorhergehenden Ansprüche in Kontakt gebracht wird und die Bindung von Antikörpern aus der biologischen Probe an diese Polypeptide erfolgen kann, wobei durch den Nachweis von Antikörpern gegen eines dieser Polypeptide eine Zöliakie oder Dermatitis herpetiformis diagnostiziert wird.

8. Verfahren nach Anspruch 7, **dadurch gekennzeichnet, dass** ein Gemisch aus mindestens zwei verschiedenen Polypeptiden nach einem der Ansprüche 1 bis 6 verwendet wird.

9. Verfahren nach Anspruch 7 oder 8, **dadurch gekennzeichnet, dass** die Bindung der Antikörper mit einem Immunfluoreszenztest, Microarray, ELISA, Lumineszenztest, Blot, Radioimmuntest, Western Blot oder Dot Blot nachgewiesen wird.

10. Testsatz zum Nachweis von Antikörpern, **dadurch gekennzeichnet, dass** er ein Polypeptid nach einem der Ansprüche 1 bis 6 umfasst.

11. Polypeptid nach einer der Ansprüche 1 bis 6 zur Verwendung bei der Therapie von Zöliakie und/oder Dermatitis herpetiformis, **dadurch gekennzeichnet, dass** Blut oder Plasma eines Patienten mit einem Polypeptid nach einem der Ansprüche 1 bis 7 in Kontakt gebracht wird und die Bindung der krankheitsassoziierten Antikörper aus dem Blut oder Plasma an das Polypeptid erfolgt, wobei das Antikörper-abgereicherte Blut oder Plasma zur Reinfusion an den Patienten geeignet ist.

12. Pharmazeutische Zusammensetzung, **dadurch gekennzeichnet, dass** sie ein Polypeptid nach einem der Ansprüche 1 bis 6 umfasst.

13. Verwendung eines Polypeptids nach einem der Ansprüche 1 bis 6 für die in vitro-Diagnose von Zöliakie und/oder Dermatitis herpetiformis.

14. Verwendung eines Polypetids nach einem der Ansprüche 1-6 zur Herstellung eines Medikaments für die Therapie von Zöliakie und/oder Dermatitis herpetiformis.

15. Nukleinsäure, welche für ein oder mehrere Polypeptide nach einem der Ansprüche 1 bis 6 kodiert.

## Claims

1. A polypeptide comprising one or several peptides according to SEQ ID NO: 2 and one or several peptides according to SEQ ID NO: 3, wherein up to 30% of the amino acids may be deleted or substituted by conservative amino acid substitutions, and/or SEQ ID NO: 2 and SEQ ID NO: 3 may partially overlap, **characterized in that** it comprises one or several polypeptides consisting of at least 14, at least 15, or at least 16 consecutive amino acids according to SEQ ID NO: 4 or comprises one or several polypeptides according to SEQ ID NO: 4.

2. The polypeptide of claim 1, comprising three polypeptides according to SEQ ID NO: 4.

3. The polypeptide of one of the preceding claims, comprising one or several polypeptides according to SEQ ID NO: 7.

4. A polypeptide of one of the preceding claims in form of a polymer, comprising 2 to 20 polypeptides.

5. The polypeptide of one of the preceding claims, **characterized in that** it is produced recombinantly or with common biochemical means of polypeptide synthesis.

6. The polypeptide of one of the preceding claims, **characterized in that** it is bound to a reporter molecule or to a solid phase.

7. A method for the diagnosis of celiac disease and/or dermatitis herpetiformis, **characterized in that** a liquid biological sample from a patient is contacted with one or several polypeptides according to one of the preceding claims and that the binding of antibodies from the biological sample to said polypeptides can take place, wherein celiac disease or dermatitis herpetiformis is diagnosed by detection of antibodies against one of said polypeptides.

8. The method of claim 7, **characterized in that** a mixture of at least two different polypeptides of one of claims 1 to 6 is used.

9. The method of claims 7 or 8, **characterized in that** the binding of the antibodies is detected using an immunofluorescence test, microarray, ELISA, luminescence test, blot, radioimmunoassay, Western Blot or dot blot.

10. A test kit for the detection of antibodies, **characterized in that** it comprises a polypeptide according to one of claims 1 to 6.

11. Polypeptide of one of claims 1 to 6 for use in a therapy of celiac disease and/or dermatitis herpetiformis, **characterized in that** blood or plasma of a patient is contacted with a polypeptide of one of claims 1 to 7 and the binding of the disease associated antibodies from the blood or plasma takes place, wherein the antibody depleted blood or plasma is suitable for reinfusion to the patient.

12. A pharmaceutical composition, **characterized in that** it comprises a polypeptide of one of claims 1 to 6.

13. Use of a polypeptide of one of claims 1 to 6 for the in vitro diagnosis of celiac disease and/or dermatitis herpetiformis.

14. Use of a polypeptide of one of claims 1-6 for the manufacture of a medicament for the therapy of celiac disease and/or dermatitis herpetiformis.

15. A nucleic acid encoding one or several polypeptides according to one of claims 1 to 6.

## Revendications

1. Polypeptide, qui comprend un ou plusieurs peptides respectant la SEQ ID n° 2 et un ou plusieurs peptides respectant la SEQ ID n° 3, dans lequel respectivement jusqu'à 30 % des acides aminés peuvent être supprimés ou substitués par le biais d'une substitution conservatrice d'acides aminés et/ou la SEQ ID n° 2 et la SEQ ID n° 3 peuvent se chevaucher en partie, **caractérisé en ce qu'**il comprend un ou plusieurs polypeptides constitués d'au moins 14, d'au moins 15 ou d'au moins 16 acides aminés successifs dans la SEQ ID n° 4 ou comprend un ou plusieurs polypeptides respectant la SEQ ID n° 4.

2. Polypeptide selon la revendication 1, comprenant trois polypeptides respectant la SEQ ID n° 4.

3. Polypeptide selon l'une quelconque des revendications précédentes, comprenant un ou plusieurs polypeptides respectant la SEQ ID n° 7.

4. Polypeptide selon l'une quelconque des revendications précédentes sous la forme d'un polymère, comprenant 2 à 20 polypeptides.

5. Polypeptide selon l'une quelconque des revendications précédentes, **caractérisé en ce qu'**il est produit de manière recombinante ou avec des moyens biochimiques traditionnels de la synthèse polypeptidique.

6. Polypeptide selon l'une quelconque des revendications précédentes, **caractérisé en ce qu'**il est lié à une molécule rapporteur ou à une phase solide.

7. Procédé de diagnostic d'une maladie coeliaque et/ou d'une dermatite herpétiforme, **caractérisé en ce qu'**un échantillon biologique liquide d'un patient est mis en contact avec un ou plusieurs polypeptides selon l'une quelconque des revendications précédentes et **en ce que** la liaison d'anticorps provenant de l'échantillon biologique peut s'effectuer sur ces polypeptides, dans lequel une maladie coeliaque ou une dermatite herpétiforme est diagnostiquée par la détection d'anticorps dirigés contre l'un de ces polypeptides.

8. Procédé selon la revendication 7, **caractérisé en ce que** l'on utilise un mélange d'au moins deux polypeptides différents selon l'une quelconque des revendications 1 à 6.

9. Procédé selon la revendication 7 ou 8, **caractérisé en ce que** la liaison des anticorps est détectée avec un test d'immunofluorescence, un test sur microréseau, un test ELISA, un test par luminescence, un buvardage, un test radio-immunologique, un buvardage de western ou une hybridation sur tache.

10. Trousse d'essai pour la détection d'anticorps, **caractérisée en ce qu'**elle comprend un polypeptide selon l'une quelconque des revendications 1 à 6.

11. Polypeptide selon l'une quelconque des revendications 1 à 6 pour l'utilisation dans la thérapie d'une maladie coeliaque et/ou d'une dermatite herpétiforme, **caractérisé en ce que** du sang ou du plasma d'un patient est mis en contact avec un polypeptide selon l'une quelconque des revendications 1 à 7 et **en ce que** la liaison des anticorps associés à la maladie et provenant du sang ou du plasma s'effectue sur le polypeptide, le sang ou le plasma appauvri en anticorps demeurant approprié pour une reperfusion vers le patient.

12. Composition pharmaceutique, **caractérisée en ce qu'**elle comprend un polypeptide selon l'une quelconque des revendications 1 à 6.

13. Utilisation d'un polypeptide selon l'une quelconque des revendications 1 à 6 pour le diagnostic in vitro d'une maladie coeliaque et/ou d'une dermatite herpétiforme.

14. Utilisation d'un polypeptide selon l'une quelconque des revendications 1 à 6 pour la fabrication d'un médicament pour la thérapie d'une maladie coeliaque et/ou d'une dermatite herpétiforme.

15. Acide nucléique codant pour un ou plusieurs polypeptides selon l'une quelconque des revendications 1 à 6.


### SEQUENCE LISTING

<110>
<120> Verfahren und Immunabsorbentien zur spezifischen Detektion und Absorption Zöliakie- und Dermatitis herpetiformis assoziierter Antikörper.
<130> 10 2007 025 291.0
<140> unknown
   <141> 2007-05-30
<160> 11
<170> PatentIn version 3.3
<210> 1
   <211> 16
   <212> PRT
   <213> artificial
<220>
   <223> Peptide sequence D-2 according to Aleanzi et al. 2001. Clin Chem. 47:2023-2028
<400> 1
   Gln Pro Glu Gln Pro Gln Gln Ser Phe Pro Glu Gln Glu Arg Pro Phe
   1 5 10 15
<210> 2
   <211> 9
   <212> PRT
   <213> artificial
<220>
   <223> Nonapeptide sequence according to Schwertz et al. 2004. Clinical Chemistry 50:2370-2375
<400> 2
   Pro Leu Gln Pro Glu Gln Pro Phe Pro
   1 5
<210> 3
   <211> 9
   <212> PRT
   <213> artificial
<220>
   <223> Nonapeptide sequence according to Schwertz et al. 2004. Clinical Chemistry 50:2370-2375
<400> 3
   Pro Glu Gln Leu Pro Gln Phe Glu Glu
   1 5
<210> 4
   <211> 17
   <212> PRT
   <213> artificial
<220>
   <223> synthetic fusion peptide of SEQ ID NO 2 and SEQ ID NO 3
<400> 4
   Pro Leu Gln Pro Glu Gln Pro Phe Pro Glu Gln Leu Pro Gln Phe Glu
   1 5 10 15
   Glu
<210> 5
   <211> 198
   <212> DNA
   <213> artificial
<220>
   <223> Three copies of the linker DNA made by hybridization of DNA oligonucleotides according to SEQ ID NO 10 and SEQ ID NO 11 are ligated in AdeI linearized pET24e vector DNA. The resulting coding sequence for the protein according to SEQ ID NO 7 is shown.
<400> 5
   atggctcaca tgccgctgca gcctgaacaa ccatttcccg aacaactgcc gcagtttgaa    60
   gaaatgccgc tgcagcctga acaaccattt cccgaacaac tgccgcagtt tgaagaaatg   120
   ccgctgcagc ctgaacaacc atttcccgaa caactgccgc agtttgaaga aatggtgcac   180
   caccaccacc accactga                                                  198
<210> 6
   <211> 5401
   <212> DNA
   <213> E.coli
<223> pET24e-GAF(3X) expression vector used as GAF(3X) peptide expression construct.
<400> 6
   ggggaattgt gagcggataa caattcccct ctagaaataa ttttgtttaa ctttaagaag    60
   gagatatacc atggctcaca tgccgctgca gcctgaacaa ccatttcccg aacaactgcc   120
   gcagtttgaa gaaatgccgc tgcagcctga acaaccattt cccgaacaac tgccgcagtt   180
   tgaagaaatg ccgctgcagc ctgaacaacc atttcccgaa caactgccgc agtttgaaga   240
   aatggtgcac caccaccacc accactgaga tccggctgct aacaaagccc gaaaggaagc   300
   tgagttggct gctgccaccg ctgagcaata actagcataa ccccttgggg cctctaaacg   360
   ggtcttgagg ggttttttgc tgaaaggagg aactatatcc ggattggcga atgggacgcg   420
   ccctgtagcg gcgcattaag cgcggcgggt gtggtggtta cgcgcagcgt gaccgctaca   480
   cttgccagcg ccctagcgcc cgctcctttc gctttcttcc cttcctttct cgccacgttc   540
   gccggctttc cccgtcaagc tctaaatcgg gggctccctt tagggttccg atttagtgct   600
   ttacggcacc tcgaccccaa aaaacttgat tagggtgatg gttcacgtgg gccatcgccc   660
   tgatagacgg tttttcgccc tttgacgttg gagtccacgt tctttaatag tggactcttg   720
   ttccaaactg gaacaacact caaccctatc tcggtctatt cttttgattt ataagggatt 780
   ttgccgattt cggcctattg gttaaaaaat gagctgattt aacaaaaatt taacgcgaat 840
   tttaacaaaa tattaacgtt tacaatttca ggtggcactt ttcggggaaa tgtgcgcgga 900
   acccctattt gtttattttt ctaaatacat tcaaatatgt atccgctcat gaattaattc 960
   ttagaaaaac tcatcgagca tcaaatgaaa ctgcaattta ttcatatcag gattatcaat 1020
   accatatttt tgaaaaagcc gtttctgtaa tgaaggagaa aactcaccga ggcagttcca 1080
   taggatggca agatcctggt atcggtctgc gattccgact cgtccaacat caatacaacc 1140
   tattaatttc ccctcgtcaa aaataaggtt atcaagtgag aaatcaccat gagtgacgac 1200
   tgaatccggt gagaatggca aaagtttatg catttctttc cagacttgtt caacaggcca 1260
   gccattacgc tcgtcatcaa aatcactcgc atcaaccaaa ccgttattca ttcgtgattg 1320
   cgcctgagcg agacgaaata cgcgatcgct gttaaaagga caattacaaa caggaatcga 1380
   atgcaaccgg cgcaggaaca ctgccagcgc atcaacaata ttttcacctg aatcaggata 1440
   ttcttctaat acctggaatg ctgttttccc ggggatcgca gtggtgagta accatgcatc 1500
   atcaggagta cggataaaat gcttgatggt cggaagaggc ataaattccg tcagccagtt 1560
   tagtctgacc atctcatctg taacatcatt ggcaacgcta cctttgccat gtttcagaaa 1620
   caactctggc gcatcgggct tcccatacaa tcgatagatt gtcgcacctg attgcccgac 1680
   attatcgcga gcccatttat acccatataa atcagcatcc atgttggaat ttaatcgcgg 1740
   cctagagcaa gacgtttccc gttgaatatg gctcataaca ccccttgtat tactgtttat 1800
   gtaagcagac agttttattg ttcatgacca aaatccctta acgtgagttt tcgttccact 1860
   gagcgtcaga ccccgtagaa aagatcaaag gatcttcttg agatcctttt tttctgcgcg 1920
   taatctgctg cttgcaaaca aaaaaaccac cgctaccagc ggtggtttgt ttgccggatc 1980
   aagagctacc aactcttttt ccgaaggtaa ctggcttcag cagagcgcag ataccaaata 2040
   ctgtccttct agtgtagccg tagttaggcc accacttcaa gaactctgta gcaccgccta 2100
   catacctcgc tctgctaatc ctgttaccag tggctgctgc cagtggcgat aagtcgtgtc 2160
   ttaccgggtt ggactcaaga cgatagttac cggataaggc gcagcggtcg ggctgaacgg 2220
   ggggttcgtg cacacagccc agcttggagc gaacgaccta caccgaactg agatacctac 2280
   agcgtgagct atgagaaagc gccacgcttc ccgaagggag aaaggcggac aggtatccgg 2340
   taagcggcag ggtcggaaca ggagagcgca cgagggagct tccaggggga aacgcctggt 2400
   atctttatag tcctgtcggg tttcgccacc tctgacttga gcgtcgattt ttgtgatgct 2460
   cgtcaggggg gcggagccta tggaaaaacg ccagcaacgc ggccttttta cggttcctgg 2520
   ccttttgctg gccttttgct cacatgttct ttcctgcgtt atcccctgat tctgtggata 2580
   accgtattac cgcctttgag tgagctgata ccgctcgccg cagccgaacg accgagcgca 2640
   gcgagtcagt gagcgaggaa gcggaagagc gcctgatgcg gtattttctc cttacgcatc 2700
   tgtgcggtat ttcacaccgc atatatggtg cactctcagt acaatctgct ctgatgccgc 2760
   atagttaagc cagtatacac tccgctatcg ctacgtgact gggtcatggc tgcgccccga 2820
   cacccgccaa cacccgctga cgcgccctga cgggcttgtc tgctcccggc atccgcttac 2880
   agacaagctg tgaccgtctc cgggagctgc atgtgtcaga ggttttcacc gtcatcaccg 2940
   aaacgcgcga ggcagctgcg gtaaagctca tcagcgtggt cgtgaagcga ttcacagatg 3000
   tctgcctgtt catccgcgtc cagctcgttg agtttctcca gaagcgttaa tgtctggctt 3060
   ctgataaagc gggccatgtt aagggcggtt ttttcctgtt tggtcactga tgcctccgtg 3120
   taagggggat ttctgttcat gggggtaatg ataccgatga aacgagagag gatgctcacg 3180
   atacgggtta ctgatgatga acatgcccgg ttactggaac gttgtgaggg taaacaactg 3240
   gcggtatgga tgcggcggga ccagagaaaa atcactcagg gtcaatgcca gcgcttcgtt 3300
   aatacagatg taggtgttcc acagggtagc cagcagcatc ctgcgatgca gatccggaac 3360
   ataatggtgc agggcgctga cttccgcgtt tccagacttt acgaaacacg gaaaccgaag 3420
   accattcatg ttgttgctca ggtcgcagac gttttgcagc agcagtcgct tcacgttcgc 3480
   tcgcgtatcg gtgattcatt ctgctaacca gtaaggcaac cccgccagcc tagccgggtc 3540
   ctcaacgaca ggagcacgat catgcgcacc cgtggggccg ccatgccggc gataatggcc 3600
   tgcttctcgc cgaaacgttt ggtggcggga ccagtgacga aggcttgagc gagggcgtgc 3660
   aagattccga ataccgcaag cgacaggccg atcatcgtcg cgctccagcg aaagcggtcc 3720
   tcgccgaaaa tgacccagag cgctgccggc acctgtccta cgagttgcat gataaagaag 3780
   acagtcataa gtgcggcgac gatagtcatg ccccgcgccc accggaagga gctgactggg 3840
   ttgaaggctc tcaagggcat cggtcgagat cccggtgcct aatgagtgag ctaacttaca 3900
   ttaattgcgt tgcgctcact gcccgctttc cagtcgggaa acctgtcgtg ccagctgcat 3960
   taatgaatcg gccaacgcgc ggggagaggc ggtttgcgta ttgggcgcca gggtggtttt 4020
   tcttttcacc agtgagacgg gcaacagctg attgcccttc accgcctggc cctgagagag 4080
   ttgcagcaag cggtccacgc tggtttgccc cagcaggcga aaatcctgtt tgatggtggt 4140
   taacggcggg atataacatg agctgtcttc ggtatcgtcg tatcccacta ccgagatatc 4200
   cgcaccaacg cgcagcccgg actcggtaat ggcgcgcatt gcgcccagcg ccatctgatc 4260
   gttggcaacc agcatcgcag tgggaacgat gccctcattc agcatttgca tggtttgttg   4320
   aaaaccggac atggcactcc agtcgccttc ccgttccgct atcggctgaa tttgattgcg   4380
   agtgagatat ttatgccagc cagccagacg cagacgcgcc gagacagaac ttaatgggcc   4440
   cgctaacagc gcgatttgct ggtgacccaa tgcgaccaga tgctccacgc ccagtcgcgt   4500
   accgtcttca tgggagaaaa taatactgtt gatgggtgtc tggtcagaga catcaagaaa   4560
   taacgccgga acattagtgc aggcagcttc cacagcaatg gcatcctggt catccagcgg   4620
   atagttaatg atcagcccac tgacgcgttg cgcgagaaga ttgtgcaccg ccgctttaca   4680
   ggcttcgacg ccgcttcgtt ctaccatcga caccaccacg ctggcaccca gttgatcggc   4740
   gcgagattta atcgccgcga caatttgcga cggcgcgtgc agggccagac tggaggtggc   4800
   aacgccaatc agcaacgact gtttgcccgc cagttgttgt gccacgcggt tgggaatgta   4860
   attcagctcc gccatcgccg cttccacttt ttcccgcgtt ttcgcagaaa cgtggctggc   4920
   ctggttcacc acgcgggaaa cggtctgata agagacaccg gcatactctg cgacatcgta   4980
   taacgttact ggtttcacat tcaccaccct gaattgactc tcttccgggc gctatcatgc   5040
   cataccgcga aaggttttgc gccattcgat ggtgtccggg atctcgacgc tctcccttat   5100
   gcgactcctg cattaggaag cagcccagta gtaggttgag gccgttgagc accgccgccg   5160
   caaggaatgg tgcatgcaag gagatggcgc ccaacagtcc cccggccacg gggcctgcca   5220
   ccatacccac gccgaaacaa gcgctcatga gcccgaagtg gcgagcccga tcttccccat   5280
   cggtgatgtc ggcgatatag gcgccagcaa ccgcacctgt ggcgccggtg atgccggcca   5340
   cgatgcgtcc ggcgtagagg atcgagatct cgatcccgcg aaattaatac gactcactat   5400
   a                                                                   5401
<210> 7
   <211> 65
   <212> PRT
   <213> artificial
<220>
   <223> Recombinant fusion protein containing a C-terminal hexa-histidine-Tag for protein purification and three copies of the Gliadin analogue synthetic sequence according to SEQ ID NO 4 interspaced with a single methionine residue.
<400> 7
<210> 8
   <211> 36
   <212> DNA
   <213> artificial
<220>
   <223> Chemically synthesized DNA oligonucleotide primer used to amplify pET24e-fragment by PCR.
<400> 8
   ctcacatggt gcaccaccac caccaccact gagatc 36
<210> 9
   <211> 44
   <212> DNA
   <213> artificial
<220>
   <223> Chemically synthesized DNA oligonucleotide primer used to amplify the pET24e-fragment by PCR.
<400> 9
   gtgcaccatg tgagccatgg tatatctcct tcttaaagtt aaac 44
<210> 10
   <211> 54
   <212> DNA
   <213> artificial
<220>
   <223> 5'-phosphorylated chemically synthesized sense DNA oligonucleotide that hybridizes with DNA oligonucleotide according to SEQ ID NO 11 to form the linker to be inserted in AdeI linearized pET24e.
<400> 10
   ccgctgcagc ctgaacaacc atttcccgaa caactgccgc agtttgaaga aatg 54
<210> 11
   <211> 54
   <212> DNA
   <213> artificial
<220>
   <223> 5'-phosphorylated chemically synthesized antisense DNA oligonucleotide that hybridizes with DNA oligonucleotide according to SEQ ID NO 10 to form the linker to be inserted in AdeI linearized pET24e.
<400> 11
   ttcttcaaac tgcggcagtt gttcgggaaa tggttgttca ggctgcagcg gcat 54